# EUROPEAN PATENT SPECIFICATION

(11) **EP 1 379 293 B1**
(45) Date of publication and mention of the grant of the patent: **18.02.2009**
(21) Application number: 02732636.2
(22) Date of filing: 17.04.2002
(51) Int. Cl.: A61M 1/00

(54) **BLOOD COLLECTION APPARATUS**
BLUTSAMMELVORRICHTUNG
APPAREIL DE PRELEVEMENT SANGUIN

(30) Priority: 17.04.2001 GB 0109443
(43) Date of publication of application: 14.01.2004
(60) Divisional application: 06025378.8
(73) Proprietor: SUMMIT MEDICAL LIMITED, Bourton on the Water, Gloucestershire GL54 2HQ (GB)
(72) Inventor: FOSTER, David, Woodstock, Oxforshire OX20 1QH (GB)
(74) Representative: Hughes, Andrea Michelle
(86) International application number: PCT/EP2002/004267
(87) International publication number: WO 2002/083199

(56) References cited:
- DE-A- 19 507 580
- US-A- 3 933 439
- US-A- 4 141 361
- US-A- 4 397 643
- US-A- 5 102 404
- US-A- 5 658 271
- US-A- 5 788 661

## Description

The present invention relates to an apparatus for collecting blood from a patient, and, in particular, apparatus for use in autologous blood transfusion. The apparatus may also be used for wound drainage.

During surgery, blood is drawn from the operation site of the patient.

In some cases, the blood needs to be removed from the site to allow the site to be clearly seen and accessed during the operation. Also, during and after the operation, the wound needs to drained of blood.

The patient may bleed profusely during and after an operation and, after such blood loss, will need to undergo a blood transfusion. Known ways of providing blood transfusions are Homologous Blood Transfusion in which blood is collected from donors other than the recipient (often referred to as Allogenic transfusions), and Autologous Blood Transfusion, wherein blood is collected from the patient during the operation or shortly after the operation and the same blood, collected from the patient, is then reinfused into the patient.

There are a number of risks associated with Homologous Blood transfusion. Infectious agents, e.g. hepatitis, CJD, HIV, Cytomegalovirus and other viruses, can be transmitted from the donors to the patient.

Transfusion of blood from another can cause non-haemolytic febrile transfusion reactions. Other associated risks are those of alloimmunisation, post transfusion thrombocytopenia, firbonectin depletion, histamine release and transfusion induced immunosuppression. Autologous Blood Transfusion, in contrast, has a number of advantages. As the patient's own blood is used, there is a huge saving on donated blood of which more is then available for other applications. There is an improved postoperative response and the risks of infection, transfusion reaction and alloimmunisation are substantially reduced.

Furthermore, Autologous Blood Transfusion is much cheaper than Homologous Blood Transfusion and also much more convenient.

Autologous Blood Transfusion has, therefore, become extremely popular and is particularly useful in orthopedic surgery, spinal surgery, vascular surgery, cardio-vascular surgery and liver surgery.

Thus, there is a need in this field for suitable apparatus for collecting the blood from the patient.

Two main types of blood collection or blood drainage apparatus are known in the art.

One of these is a flask type collection system, wherein blood is drawn from the patient by suction into a rigid collection flask. A blood collection bag is attached to an outlet port of the collection flask by means of a connector arrangement such as a clamp and push fit connector, a clamp in combination with a continuous line to the blood bag, or an open-and-close valve. The clamp and continuous line is used to allow re-infusion to the patient (the line is very long) whilst the flask continues to collect. This has benefits for Jehovah's witnesses. Once the required amount of blood has been collected in the flask, the valve is opened and the blood is dropped down into the bag which is then sealed. The bag is marked with identification data of the patient etc. and the blood may then be either disposed of, stored or used for autologous blood transfusion.

Another type of wound drainage blood collection system is in the form of a plastic bellows type collection chamber. Before collection, the bellows is primed by compressing the chamber. Blood is then collected under the vacuum formed by the bellows, along the patient line connected to the input of bellows chamber. Again, a blood collection bag is connected to the output of the blood collection chamber by means of a valve. With the bellows type system, blood is continually passed through the chamber into the bag, during collection. Again, the bag is then removed and the blood is either disposed of, or used for autologous blood transfusion.

Generally, in wound drainage systems, the solid flask type arrangements provide a high vacuum and are used, therefore, to draw blood from less sensitive wound regions. In more sensitive regions, e.g. in areas around sensitive nerves, the lower vacuum bellows type system is used. Below is a table showing various types of surgery for which high and/or low vacuum drainage is suitable.

| | **High Vacuum** | **Low Vacuum** |
|---|---|---|
| **Orthopaedics** | | |
| Knee | Yes | Yes |
| Hip | Yes | Yes |
| Spine | Yes | Yes |

| **General** | | |
|---|---|---|
| Mastectomy | Yes | Yes |
| Axillary Lymph | No | Yes |
| Abdomina | No | Yes |
| Tumour | Yes | No |
| Arterial | No | Yes |
| Hernia | Yes | Yes |

| **Gynaecology** | | |
|---|---|---|
| Vagina Plastics | No | Yes |
| Cystocele | Yes - only 150ml version | No |
| Caesarean | No | Yes |

| **ORL Surgery** | | |
|---|---|---|
| Thyroidectomy | No | Yes |
| Parotidectomy | Yes | No |
| Laryngectomy | Yes | No |

| **Plastic Surgery** | | |
|---|---|---|
| Hand | Yes | Yes |
| Face Lift | Yes | No |
| Breast | Yes | No |

| **Urology** | | |
|---|---|---|
| Nephroctomy | No | Yes |

| **Cardio Vascular** | | |
|---|---|---|
| Plexus | No | Yes |
| Trepanation | No | Yes |
| Cervical HNP | No | Yes |
| Sub dural Haematoma | No | Yes |
| Intra cranial | No | Yes |

| **Dental** | | |
|---|---|---|
| Jaws | Yes | No |
| Teeth Root | Yes | No |

Similarly, in autologous blood transfusion systems, the blood is collected, generally, by means of either a flask collection system or a bellows collection system at low level vacuum.

As in autologous blood transfusion, the blood is reused by reinfusion into the patient, there are, naturally, very stringent criteria as to the conditions under which the blood is collected, including the types of material used, the operating conditions, pressure applied, time over which the blood is collected, etc.

For example, the maximum pressure under which the blood should be collected is 100 mmHg. Pressures higher than this damage the blood cells and this means that the blood can no longer be reinfused into the patient.

The regulations require that in autologous blood transfusion, the maximum permissible blood collection time is six hours. If blood continues to be collected in the flask beyond that period, the blood cells can deteriorate (since the age of the blood affects its ability to perform) and the blood cannot be reused by reinfusion. Research shows that, generally, 500 to 700ml of blood are collected in any six-hour period. At the end of this period, the bag must be sealed and the blood reinfused into the patient.

However, during certain operations, for example during knee operations, the patient loses around 1 litre of blood and there is, therefore, a need to keep collecting blood from the wound or operation site beyond this six-hour period.

The blood to be collected after the six-hour period can be collected in a second bag but, unless the conditions are completely sterile, on attachment of the second bag, this second lot of blood is not suitable for reinfusion and must be disposed of. This blood is, therefore, wasted.

As mentioned above, there are, essentially, two standard types of product currently in use for blood collection, particularly for autologous blood transfusion.

One of these is a rigid flask type system manufactured by Duxbury Scientific and described in, for example, US Patent No. 5,628,726.

Another flask type collection apparatus is manufactured by Stryker Corp. and is described, for example, in US Patents No. 5,830,198, and 5,645,540.

With the flask type arrangements, which comprise a rigid plastic flask having an inlet port and an outlet port, a vacuum is applied to the flask, via a vacuum port. Blood is then drawn from the patient, along the patient line, connected to the input of the flask. The blood is drawn into the flask, through a filter, and collects in the flask. The outlet port, which is connected to a blood collection bag, is then opened and the blood which is collected in the flask is dumped down' into the bag, for retransfusion or disposal.

The flask arrangements provide a continuous constant vacuum which means that they can be used inter-operatively, as well as post-operatively.

As discussed above, it is only permissible to collect blood in a blood collection bag over a maximum period of six hours.

If more blood is to be collected, this needs to be collected in a second bag.

To simplify this, Duxbury Scientific have produced a flask system called Betatrans^{®}. The Betatrans^{®} has two outlet ports, each having a filter sock, connected to the port inside of the collection flask, and an open-and-close valve at the outlet side, to which a blood collection bag is connected.

For the first collection period, up to six hours, blood is collected in the flask and dropped down into one of the bags, while the valve to the other bag is kept closed. At the end of that blood collection period, either when the bag is full or when the six-hour period has expired, the valve to first bag is closed and the bag is removed. The further blood is then collected in the second bag.

Although the connector valves on the Betatrans^{®} product, for connecting the blood bags, are relatively simple, they involve a click-type fit arrangement which is different from the previously and commonly used push-fit connectors.

Research has shown that these new valve connectors have not been considered as a benefit with operation room personnel, who are used to using the standard push-fit connectors, and they have not been adopted for use, widely.

Furthermore, the flask type systems are relatively cumbersome and expensive. Also, if the flask is tilted, during use, the ports can become blocked and, therefore, anti-tilt mechanisms have been provided.

Another problem is that, as described above, in the flask arrangement, the blood is first collected in the flask and then the valve between the flask and the collection bag is opened when the blood is "dumped down" from the flask into the bag. A filter sock is provided at the output port to filter the blood before it is dumped down into the bag.

However, because the blood is collected in the flask, and there is a relatively large contact area between the blood and the flask, for a relatively long period of time, the blood sees the contact with the flask as contact with a foreign body and blood clotting can result. Even though filters are provided, blood clotting can still result at the output ports, and block the ports.

One attempted solution has been to provide a filter sock in the input to the flask which filters incoming blood instead of outgoing blood. However, the problem of the large surface area of contact with the plastic flask still exists and clots can still form.

A second type of blood collection system uses a bellows type arrangement similar to those discussed above in relation to wound drainage. One such system is the Astra Tech Bellovac^{™} system.

In this system, a catheter is placed in situ and connected to the inlet tubing. To evacuate blood from the operation area, an inlet clamp is closed, the bellows are compressed and the inlet clamp is reopened. This procedure is repeated until the blood starts to flow or the bellows remain compressed. The clamps are then closed and the device is thus primed. The blood collection bag is attached to the outlet port of the bellows and is marked with the identity of the patient, the time collection started and the time (maximum six hours) when collection should be terminated. The device is then hung in an appropriate position, using the bag rail strap. The extension line is connected between the bag and the outlet port of the bellows before collection. To empty the contents of the bellows into the collection bag, the inlet clamp is closed and the outlet clamp is opened. The bellows are then compressed slowly, using the palm of the hand, and the liquid is transferred into the bag. The outlet clamp is then closed and the inlet clamp opened to continue drainage.

Such systems are much preferred by nursing stuff, as they are relatively light, simple to use and use simple push-fit connectors at the inputs and outputs.

One feature of the bellows arrangement is that the vacuum is not constant and, therefore, such collection systems can only be used post-operatively, and not intra-operatively. However, these relatively low vacuum systems can be used in more sensitive areas. The bellows type system is much simpler, lighter and less expensive than the flask system, this latter factor being important in the field of disposable devices.

The bellows system is also more accepted by operating room personnel, as they are used to using such systems, as they are used in such systems in general low vacuum wound drainage. Again, this is an important factor in surgical apparatus, where familiarity with the operation of the devices is crucial.

Another advantage is that the blood passes continuously through the bellows vacuum chamber into the collection bag and thus remains less in contact with the plastic interior surface of the collection chamber. Thus, the risk of clotting is reduced substantially.

A further advantage is that if the bellows device is tilted or laid on a bed, as often happens in practice, there is no flow-back because there is no wall suction and, also, the one-way valve within the inlet line/bellows stops any flow-back.

As mentioned above, regulations state that blood for reinfusion must be collected within a period of no more than six hours. In many operations, however, blood needs to continue to be collected beyond six hours and, to optimise the use of this blood in autologous blood transfusion, it is useful if a new blood collection bag can be attached and a new collection started.

With the known systems, however, once the first bag is removed, because the bag is full or because the six-hour collection period has expired, a second bag can be attached to the output port, but that port may well be contaminated due to exposure to air during the exchange of the bag. Also, during exchange, when the blood becomes exposed to air, the blood remaining in the valve connector part may clot.

Thus, although a new bag may be attached to the connector port to collect the remaining blood from the patient, that blood is not of sufficient quality, or the quality cannot be guaranteed, such that the blood can be reinfused. The system can, then, only be used as a wound drainage system and the blood collected in the second bag must be disposed of.

There is thus a need for an autologous blood transfusion collection system which optimises the collection of usable blood, in accordance with all of the regulations, ensuring that all safety criteria are met, whilst providing a system which is simple, easy to use and inexpensive.

Accordingly, the present invention provides a blood collection apparatus, comprising a collection vessel having a pleated, compressible body, the vessel having an inlet port adapted to be connected to a patient line for drawing blood from a patient; characterized by said apparatus further comprising first and second outlet ports, each outlet port adapted to be connected to a blood collection bag; and further comprising a first clamp provided on said first outlet port and a second clamp provided on said second outlet port for closing said first and second outlet ports.

The input and output ports are preferably adapted to be connected to the patient line and the bags, respectively, by means of a push fit connection.

The ports are all preferably provided with closure means to optionally prevent flow through the ports.

Preferably, there is provided a one-way valve at or before the inlet port and at or connected, in use, to the outlet ports to prevent flow-back of the blood.

The invention may also provide a blood collection apparatus comprising a blood collection vessel having a pleated, compressible body, an inlet port and an outlet port; where the inlet port comprises two port connections, the first adapted to be connected to a patient line through which blood is collected from the patient, and the second being adapted to be attached to a constant vacuum.

Preferred embodiments of the invention will now be described, by way of example only, with reference to the accompanying drawings.
Fig. 1 shows a schematic view of the components of the system incorporating a device of the present invention;
Figs. 2A and 2B show, in detail, a section through the inlet- and outlet ports, respectively;
Figs. 3a to 3d show the steps involved in using in an apparatus according to the present invention;
Figs. 4a - 4c show a second embodiment of the apparatus;
Fig. 5 shows an adapted inlet port, for use in a second embodiment of the present invention.

Although the system of the present invention has been described above in relation to Autologous Blood Transfusion, because it is particularly advantageous in such applications, it can, of course, also be used in a standard wound drainage application.

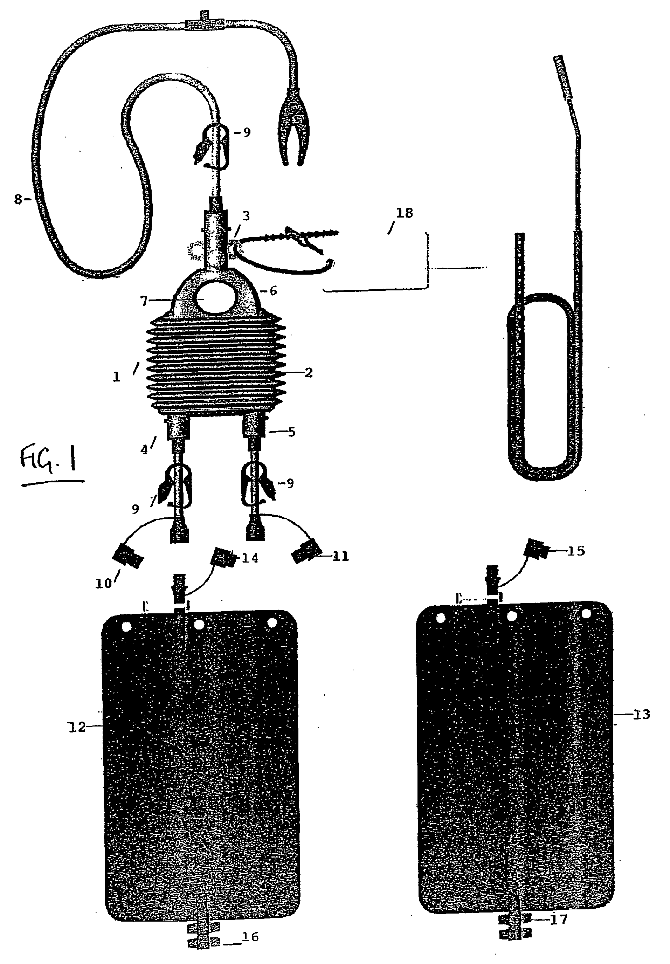

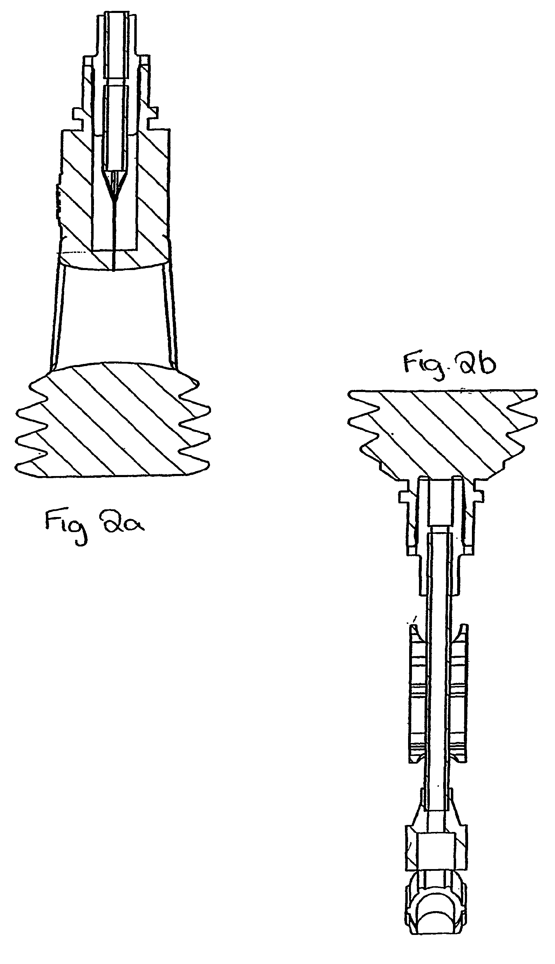

A first embodiment of the invention will now be described, with reference to figures 1 and 2.

Figure 1 shows the components of an autologous blood transfusion system incorporating the collection flask arrangement of the present invention.

Figs. 2a and 2b show in more detail the inlet and outlet ports.

The collection apparatus of the present invention comprises a collection chamber 1 having a compressible, pleated body 2 forming a bellows-type structure. An input port 3 is provided at one end of the collection device. Two output ports 4, 5 are provided at the other end.

Between the input port 3 and the main body 2, is provided a grip 6 having a thumb hole 7. The grip 6 is shaped so that the user's thumb can be easily inserted into the thumb hole and the fingers can be arranged between the two output ports 4 and 5, when the bellows are to be compressed on priming the chamber. This design makes the bellows particularly easy to prime using only one hand.

A filter is provided inside the inlet port 3. Filters may also be provided in the outlet ports 4 and 5.

The inlet side of the input port 3 is provided with one half of a push fit connection, to which a patient line 8 can be attached, by means of the other part of the push fit connection provided on the end of the patient line.

A simple clamp 9 can be provided on the patient line 8, which can be closed, by the user, to close the patient line and cut off flow along the line.

The two output ports 4, 5, are identical. Each is provided with a closure 10, 11 adapted to be push fit into the outlet end of the output port. The closures 10, 11 are preferably attached by a thin plastic strip, to the outlet port itself. Clamps 9 are also provided on the two output ports, again to close the ports, if required.

Blood collection bags 12, 13, of a known type, are provided to be attached to each of the outlet ports, by means of a matching push fit connector. The connectors are also provided with closures 14, 15, similar to closures 10, 11, for closing the inlet ports to the bags, and a clamp is provided on each inlet port, to stop flow through the port. A one-way valve may be provided at the inlet ports to the bags to prevent flow-back of blood.

Each bag also has an outlet port 16, 17, provided with closure means.

An identification tag 18 is secured to the flask, for identification purposes.

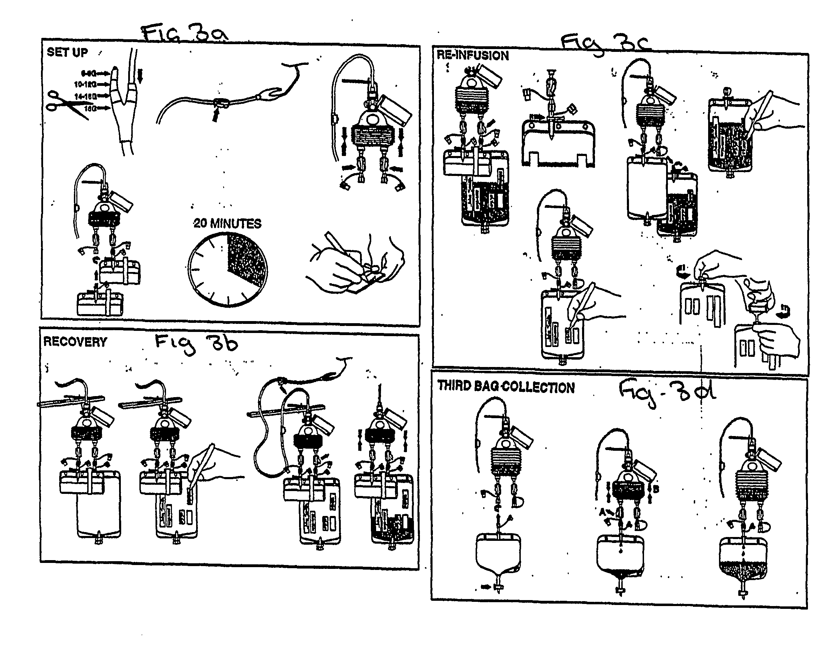

The method of use of the apparatus will now be described, with reference to figures 3a-d.

First, the person preparing the apparatus for use, or other operating room personnel must ensure that the wound is completely clean and free from any contaminates or other contraindicative substances such as a Betadine.

The components of the blood collection apparatus are provided in a sterile package, which must be opened using aseptic procedures.

The drain tubes are placed in situ. The trocar is cut off from each of the drains and catheters are connected to the inlet tubing via a universal Y connector which should be cut to fit the size of drain being used.

The patient line 8 is clamped off using clamp 9.

The bellows 2 is then primed by being completely compressed and held. Still holding the bellows compressed, the clamps 9 on the outlet ports 4, 5 are closed.

Then, a blood transfusion bag is attached to each of the outlet ports, using the push fit connectors.

Preferably, all of the clamps should remain closed for 20 minutes after, e. g., the tourniquet on the patient has been released or the wound has been closed.

The patient data, including patient's name, identity number and time should be noted on the tag 18 and also on the bags 12, 13.

The apparatus is then primed for use.

The device is then hung in an operating position, for example using the bed rail strap. The device should hang below the level of the patient, to draw the blood from the patient.

The bags are attached to the ports in a tightly rolled up state. At this time, the first transfusion bag is now unrolled.

At the appropriate time (e. g. 20 minutes after the tourniquet is released or the wound closed), blood collection begins. This time should be noted on the collection bag. As mentioned above, the maximum permissible collection time for anyone bag is 6 hours, or sooner if 600 ml of blood is collected (i. e. the bag is filled to its maximum capacity).

At the appropriate time, the clamps 9 on the patient line and the output port are opened and blood drainage begins, as the vacuum created from priming the bellows 2 draws blood along the patient line 8.

A regular check should be carried out to ensure that the bellows continues to create a vacuum and to check the volume of blood that has been collected in the transfusion bag. There may be a need to re-prime the bellows, which can be done by compressing the bellows, although in most cases this is not necessary.

At the end of the 6 hour period, or when the bag is full, the clamp on the output port is closed, the bag is removed and the closures on the output port and the bag are sealed. The bag is then ready for infusion, normally carried out on the ward.

Should the patient continue to bleed, the second bag 13 is unrolled, the clamp opened and the blood collected in the bag.

Should the patient continue to bleed further, beyond two 6 hour periods or two full collection bags, a wound drainage bag can be connected to one of the output ports, although this third collection bag may be contaminated from the already-used port and, therefore, must only be used for drainage and must not be used for retransfusion.

For reinfusion of the blood collected from the patient, the clamp to the inlet port of the bag is closed. The transfusion bag is then detached from the bellows and the closure closed, if the retransfusion is to take place during collection.

The transfusion bag is then turned upside down so that the outlet port is at the top. The outlet closure is opened and a filter is inserted. The blood can then be reinfused into the patient via a patient reinfusion line.

In the embodiments shown in Figs. 1 to 3, the inlet and outlet ports of the bellows device are provided with simple bung type closures that push-fit into the respective ports.

This bung may be made of PVC. However, there can be a problem in that after sterilisation, the PVC could release plasticisors which can compromise the force fit. Furthermore, if small moulding flaws exist in the bung, the stress produced by the push-fit can cause these flaws to split. Another problem with a simple bung and port push-fit arrangement is that, over time, air leaks can be caused between the bung and the port, causing a reduction in vacuum. The air leaks may also arise due to inaccuracies in moulding the bung.

In a preferred embodiment of the present invention, an improved cap or closure design is provided which overcomes these problems.

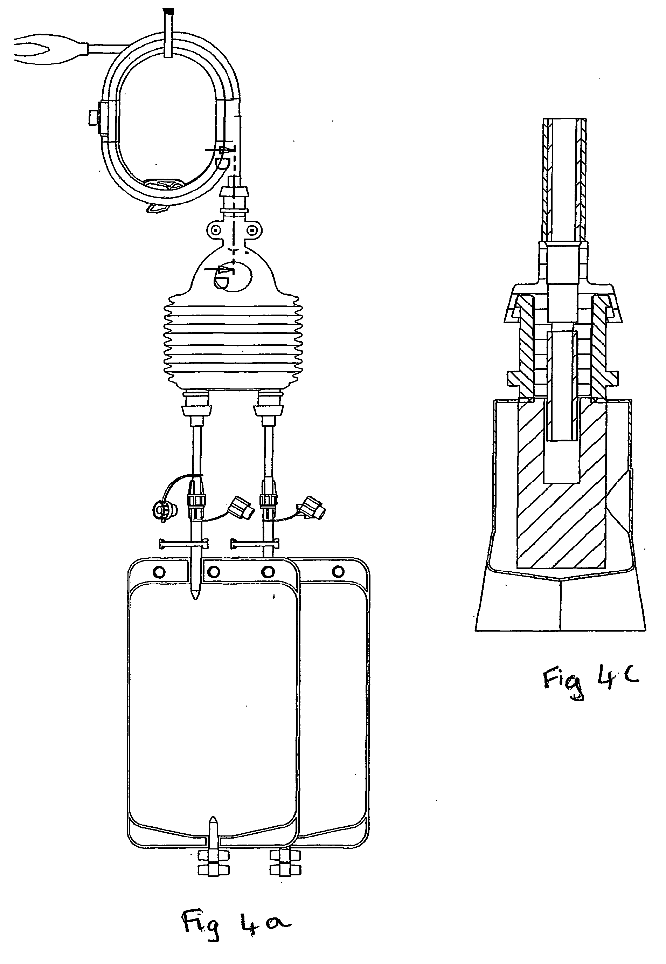

This improved design is shown in Figs. 4a to 4c.

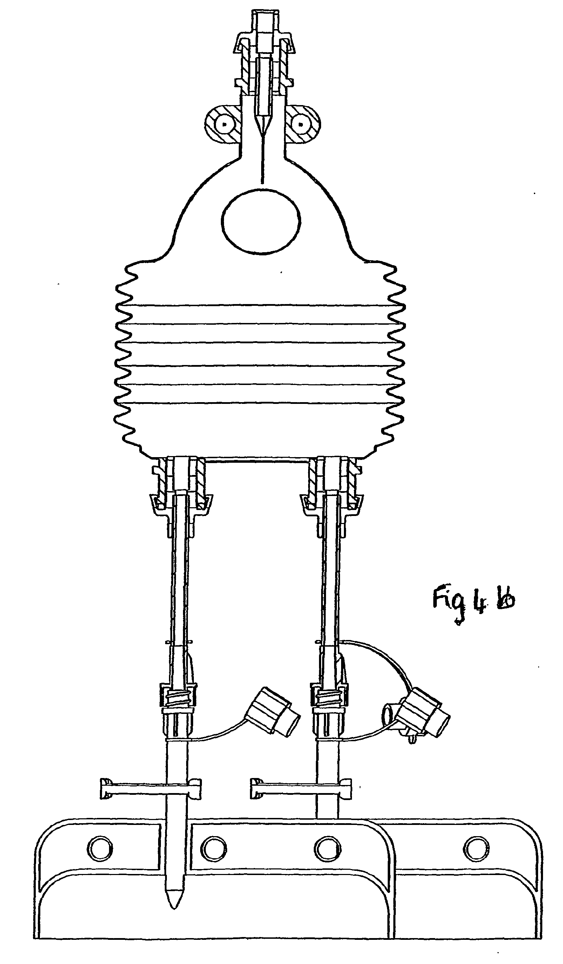

Fig. 4a shows a schematic view of the blood collection apparatus comprising the new closure arrangement. Fig. 4c is a cross-section through the closure and port arrangement at the inlet port of the bellows device. Fig. 4b is a cross-sectional view of the bellows showing, in cross section, the input and output ports.

The main features of the improved cap design are that an O-ring is provided to ensure a secure seal between the cap and the bellows port. This is compressed and held in place between the cap and the port's top surface. This top surface is free from any split or flash caused by moulding, as it forms part of the shut-off between the mould and the parison material.

With this modified closure, the port itself is also slightly modified in that it is provided with a tapered ring located at its tip. This section fits up within the cap and fits into the inner cap profile.

The cap can then be modified such that is houses all of the existing parts such as the line connector and the valves discussed previously. The profile of the cap is adapted to fit into the bore of the bellows port without over-stressing the port.

The main seal is provided between the cap, the O-ring and the bellows port. The cap snaps over the bellows port to give a strong and secure mechanical fit which overcomes any migration of plasticisors, even when PVC is used.

As mentioned above, generally, in bellows type arrangements, where the vacuum is provided by the bellows themselves, the vacuum is not constant. Therefore, such systems can only be used in postoperative blood drainage.

In a further embodiment of the invention, the inlet port to the bellows may be specially adapted to make the apparatus suitable also for intra-operative blood drainage.

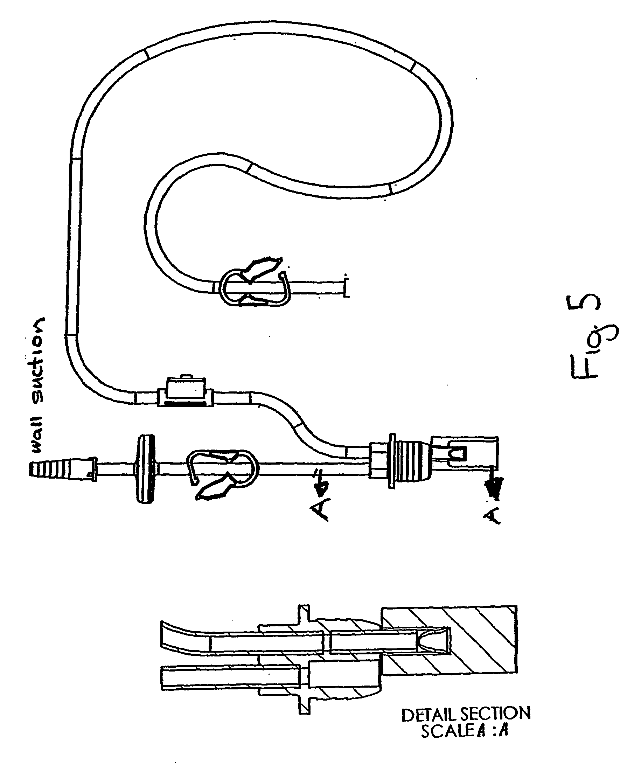

The components of the inlet port of this embodiment are shown in Fig. 5.

In this embodiment, the inlet port is adapted to have two ports, the first being a port to the patient line, as described above. The second is a port having a line adapted to be attached to a vacuum supply, e. g. a wall-mounted vacuum system, which ensures a constant vacuum through the system.

This second aspect may be combined with a 'two outlet port' collection device as described in relation to the first aspect of the invention, to allow the bellows type arrangement to be used intra-operatively as well as post-operatively, by insuring a constant vacuum.

The inlet arrangement of the second aspect of the invention is, however, also useful for other bellows type wound drainage or blood transfer devices, where a constant vacuum is required e.g. for intra-operative use, rather than the non-constant vacuum normally provided by the bellows devices.

The invention thus provides a simple, user-friendly, sterile blood collection system in which the collection of blood is optimised. Another advantage is that as the first bag is filled, the assembly tilts slightly due to the weight of the blood, and this has the effect of funnelling blood into the port. Once replaced, the same occurs with the second bag and so all blood is effectively collected.

## Claims

1. A blood collection apparatus, comprising a collection vessel having a pleated, compressible body (2), the vessel having an inlet port (3) adapted to be connected to a patient line (8) for drawing blood from a patient, **characterised by** said apparatus further comprising first and second outlet ports (4, 5), each outlet port adapted to be connected to a blood collection bag (12, 13) and further comprising a first clamp (9) provided on said first outlet port (4) and a second clamp (9) provided on said second outlet port (5) for closing said first and second outlet ports.

2. A blood collection apparatus as claimed in claim 1, wherein the inlet (3) and outlet (4, 5) ports are adapted to be connected to the patient line (8) and the bags (12, 13), respectively, by means of a push fit connection.

3. A blood collection apparatus as claimed in claim 1 or 2, provided with closure means (10, 11) to optionally prevent flow through the ports.

4. A blood collection apparatus as claimed in any preceding claim, further comprising a one-way valve at or before the inlet port (3) and at or connected, in use, to the outlet ports (4, 5) to prevent flow-back of the blood.

5. A blood collection apparatus as claimed in any preceding claim, wherein the inlet port (3) comprises two port connections, the first adapted to be connected to a patient line (8) through which blood is collected from the patient, and the second being adapted to be attached to a constant vacuum.

## Patentansprüche

1. Blutsammelvorrichtung mit einem Sammelgefäß mit einem gefalteten, komprimierbaren Körper (2), wobei das Gefäß einen Einlassstutzen (3) aufweist, der dafür eingerichtet ist, mit einer Patientenleitung (8) zum Abziehen von Blut von einem Patienten verbunden zu werden, **dadurch gekennzeichnet, dass** die Vorrichtung ferner erste und zweite Auslassstutzen (4, 5) aufweist, wobei jeder Auslassstutzen dafür eingerichtet ist, mit einem Blutsammelbeutel (12, 13) verbunden zu werden, und ferner eine erste Klammer (9), die an dem ersten Auslassstutzen (4) bereitgestellt ist, und eine zweite Klammer (9), die an dem zweiten Auslassstutzen (5) bereitgestellt ist, um die ersten und zweiten Auslassstutzen zu schließen, aufweist.

2. Blutsammelvorrichtung nach Anspruch 1, bei der die Einlass- (3) und Auslassstutzen (4, 5) dafür eingerichtet sind, jeweils mit der Patientenleitung (8) und den Beuteln (12, 13) durch eine Schiebegleitsitzverbindung bzw. eine Steckverbindung verbunden zu werden.

3. Blutsammelvorrichtung nach Anspruch 1 oder 2, die mit Abschlussmitteln (10, 11) versehen ist, um optional den Fluss durch die Stutzen zu verhindern.

4. Blutsammelvorrichtung nach einem der vorstehenden Ansprüche, die ferner ein Einwegeventil bei oder vor dem Einlassstutzen (3) und bei oder, bei Benutzung, verbunden mit den Auslassstutzen (4, 5) zum Verhindern eines Rückflusses des Bluts aufweist.

5. Blutsammelvorrichtung nach einem der vorstehenden Ansprüche, bei der der Einlassstutzen (3) zwei Stutzenverbindungen aufweist, wobei die erste dafür eingerichtet ist, mit einer Patientenleitung (8) verbunden zu werden, durch welche Blut von dem Patienten gesammelt wird, und die zweite dafür eingerichtet ist, an ein konstantes Vakuum angebracht bzw. angeschlossen zu werden.

## Revendications

1. Appareil de prélèvement sanguin, comportant un récipient de collecte ayant un corps compressible, en accordéon (2), le récipient ayant un orifice d'entrée (3) adapté pour être raccordé à une ligne de patient (8) pour prélever du sang chez un patient, **caractérisé en ce que** ledit appareil comporte également des premier et second orifices de sortie (4, 5), chaque orifice de sortie étant adapté pour être raccordé à une poche de prélèvement sanguin (12, 13) et comporte en outre un premier clamp (9) agencé sur ledit premier orifice de sortie (4) et un second clamp (9) agencé sur ledit second orifice de sortie (5) pour obturer lesdits premier et second orifices de sortie.

2. Appareil de prélèvement sanguin selon la revendication 1, dans lequel les orifices d'entrée (3) et de sortie (4, 5) sont adaptés pour être raccordés à la ligne de patient (8) et aux poches (12, 13), respectivement, au moyen d'un raccordement par emmanchement.

3. Appareil de prélèvement sanguin selon la revendication 1 ou 2, muni de moyens de fermeture (10, 11) pour empêcher facultativement un écoulement à travers les orifices.

4. Appareil de prélèvement sanguin selon l'une quelconque des revendications précédentes, comportant en outre une soupape de non-retour sur ou avant l'orifice d'entrée (3) et sur les orifices de sortie (4) ou raccordés à ceux-ci, en utilisation, pour empêcher un reflux du sang.

5. Appareil de prélèvement sanguin selon l'une quelconque des revendications précédentes, dans lequel l'orifice d'entrée (3) comporte deux raccords d'orifice, le premier étant adapté pour être raccordé à une ligne de patient (8) par l'intermédiaire de laquelle du sang est prélevé chez le patient, et le second étant adapté pour être relié à un vide constant.
